(19) Europäisches Patentamt
European Patent Office
Office européen des brevets

(11) **EP 2 770 009 B1**

(12) **EUROPÄISCHE PATENTSCHRIFT**

(45) Veröffentlichungstag und Bekanntmachung des Hinweises auf die Patenterteilung:
**06.04.2016   Patentblatt 2016/14**

(51) Int Cl.:
*C08G 65/00* (2006.01)     *C08G 65/30* (2006.01)

(21) Anmeldenummer: **14155495.6**

(22) Anmeldetag: **18.02.2014**

(54) **Verfahren zur Herstellung von Polyetherpolyolen**

Method for manufacturing polyether polyols

Procédé destiné à la fabrication de polyols de polyéther

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.02.2013  EP 13156343**

(43) Veröffentlichungstag der Anmeldung:
**27.08.2014   Patentblatt 2014/35**

(73) Patentinhaber: **Covestro Deutschland AG 51373 Leverkusen (DE)**

(72) Erfinder:
• **Lorenz, Klaus**
  **41539 Dormagen (DE)**
• **Eichmann, Marcus**
  **40547 Düsseldorf (DE)**

(74) Vertreter: **Levpat
c/o Covestro AG
Alfred-Nobel-Straße 10
40789 Monheim am Rhein (DE)**

(56) Entgegenhaltungen:
EP-A2- 0 618 251     US-A- 2 902 478
US-A- 3 190 927      US-A- 4 430 490

## Beschreibung

Die vorliegende Erfindung betrifft ein Verfahren zur Herstellung von Polyetherpolyolen durch basenkatalysierte Addition von Alkylenoxiden (Epoxiden) an bei Raumtemperatur feste Starterverbindungen mit Zerewitinoff-aktiven Wasserstoffatomen.

Polyetherpolyole basierend auf hochfunktionellen, bei Raumtemperatur festen Starterverbindungen, wie beispielsweise Zuckern, Oligo- und Polysacchariden, Zuckeralkoholen (wie beispielsweise Mannit oder Sorbit) sowie Pentaerythrit, werden in der Regel mit mehrfunktionellen Isocyanaten zu Polyurethanen umgesetzt, wobei gleichermaßen geschäumte oder massive Polyurethanwerkstoffe erhalten werden können. Spezielle Anforderungen an die Werkstoffmechanik, die Entflammbarkeit, das Durchhärteverhalten der Reaktionskomponenten oder die Hydrophilie bzw. die Hydrophobie des Werkstoffs werden in der Regel über die Struktur des Polyetherpolyols und dort wiederum durch die Wahl der Starterverbindung(en) und die Zusammensetzung der addierten Alkylenoxide erfüllt. Die Alkylenoxide können im Gemisch oder einzeln nacheinander, also blockweise, dosiert werden. Hierbei ist besonders der Einsatz von Ethylenoxid als Reinblock oder der Einsatz von hoch ethylenoxidhaltigen Blöcken neben solchen aus höheren Alkylenoxiden, wie beispielsweise Propylenoxid, hervorzuheben, da sich hiermit nicht nur Polyurethanwerkstoffe mit erhöhter Hydrophilie sondern, sofern das Ethylenoxid als Endblock eindosiert wurde, auch Polyetherpolyole mit einem erhöhten Anteil an primären Endgruppen erhalten lassen, welche dem Polyetherpolyol eine erhöhte Reaktivität gegenüber Isocyanaten verleihen.

Bei Raumtemperatur feste Starterverbindungen können Alkylenoxidadditionsreaktionen einfach zugänglich gemacht werden, indem die Alkylenoxidaddition in Gegenwart von gegenüber Alkylenoxiden unreaktiven Lösungsmitteln durchgeführt wird, wie beispielsweise in US 4,332,936 beschrieben. Im Allgemeinen ist allerdings aus Gründen der Nachhaltigkeit und Produkthygiene die Verwendung von organischen Lösungsmitteln nicht erwünscht. Weiterhin muss hierbei wertvolles Reaktorvolumen für das Lösungsmittel zur Verfügung gestellt werden.

Es können als Suspensionshilfen für die festen Starterverbindungen auch bei Raumtemperatur flüssige Starterverbindungen (Costarter) und / oder deren Alkylenoxidadditionsprodukte verwendet werden. Werden hierbei die Alkylenoxidadditionsprodukte von bei Raumtemperatur festen Starterverbindungen eingesetzt, lassen sich im Prinzip auch ausschließlich auf den hochschmelzenden Starterverbindungen basierende Polyetherpolyole lösungsmittelfrei erhalten. Solche Verfahren werden beispielsweise in FR-A 1285708 und US 3,190,927 beschrieben. Allerdings zeigen die Endprodukte häufig ein unzureichendes Lösungsvermögen für bei Raumtemperatur feste Starterverbindungen und es wird auch in diesem Fall, wie bei dem Einsatz von Lösungsmitteln, ein entsprechendes Reaktorvolumen für das Suspensionsmittel benötigt.

Wird Wasser als Suspendierungs-/ Lösungsmittel für die bei Reaktionsbedingungen festen Starterverbindungen eingesetzt, so kann die Alkylenoxidadditionsreaktion an geeigneter Stelle unterbrochen werden und das überschüssige Wasser durch Destillation abgetrennt werden. Solche Vorgehensweisen sind beispielsweise beschrieben in DE-A 1443022 und US 4,430,490. Allerdings enstehen hierbei Endprodukte mit niedrigeren Funktionalitäten durch die anteilige Reaktion des als Suspendierungs- und Lösungsmittel verwendeten Wassers bei der Alkylenoxidaddition. Weiterhin ist die Reaktion bei diesen so genannten Wasserverfahren weniger gut kontrollierbar als bei der Verwendung anderer Costarter. Die entstehenden glykolhaltigen Abwässer müssen entweder aufgereinigt werden oder deren Glykolgehalt bei Rückführung in den Prozess auf einen konstanten Wert eingestellt werden. Ebenso bedeutet die zwischenzeitliche Abdestillation des unreagierten Wassers einen zusätzlichen Zeit- und Energieaufwand.

Es wäre daher wünschenswert die Alkylenoxidadditionsreaktion möglichst in Abwesenheit von gegenüber Alkylenoxiden unreaktiven Lösungsmitteln und anderen Suspendierhilfen bei den für basenkatalysierte Alkylenoxidadditionsreaktionen üblichen angewandten Reaktionstemperaturen von 70 bis 180 °C durchzuführen. Bei der Herstellung von blockweise aufgebauten Polyetherpolyolen mit reinen Ethylenoxidblöcken bzw. solchen mit einem Anteil an Oxyethyleneinheiten von größer oder gleich 75 Gew.-% erweist es sich in Abwesenheit von Lösungsmitteln jedoch als schwierig ausgehend von festen Starterverbindungen klare und homogene Endprodukte zu erhalten.

Die im Stand der Technik offenbarten Verfahren bieten für die geschilderten Probleme mit bei Raumtemperatur festen Startern, insbesondere bei blockweise aufgebauten Polyetherpolyolen mit einem Gesamtgehalt an Oxyethyleneinheiten zwischen 5 und 85 Gew.-% bezogen auf die Masse aller Oxyalkyleneinheiten, keine befriedigende Lösung.

Aufgabe der vorliegenden Erfindung war es daher ein Verfahren zur Herstellung von Polyetherpolyolen auf Basis von bei Raumtemperatur festen Starterverbindungen zu finden, wobei die erhaltenen Polyetherpolyole aus Blöcken mit einem Gehalt an Oxyethyleneinheiten von größer oder gleich 75 Gew.-% aufgebaut sind. Der Einsatz von Lösungsmitteln und größeren Mengen von bei Raumtemperatur flüssigen Costartern sollte hierbei vermieden werden und es sollten im Ergebnis keine Trübungen sowie Inhomogenitäten in den Endprodukten auftreten.

Überraschenderweise konnte die Aufgabe gelöst werden durch ein Verfahren gemäß Ansprunch 1 zur Herstellung von Polyetherpolyolen mit n Blöcken $E_n$, jeweils umfassend wenigstens 75 Gew.-% Oxyethyleneinheiten, und m von $E_n$ verschiedenen Blöcken Am, mit n und m als natürlichen Zahlen von 1 bis 10, wobei bei dem Verfahren wenigstens eine Starterverbindung mit zumindest einem Zerewitinoffaktiven Wasserstoffatom unter Zusatz eines basischen Katalysators mit Ethylenoxid sowie Propylenoxid umgesetzt wird wobei Keine Lösemittel verwendet werden und die wenigstens eine Starterverbindung einen Schmelzpunkt von $\geq$ 40 °C aufweist und wobei die Umsetzung mit Ethylenoxid während der Dosierung des Blockes oder der Blöcke $E_n$ unter den Bedingungen der folgenden Ungleichung erfolgt:

$$\frac{T_n * x(cat) * t_n}{x_n(EO)} > 2{,}9$$

$$\text{in } [°C * h] \qquad\qquad (1)$$

wobei

$T_n$, als mittlere Reaktionstemperatur in °C während der Dosierung des Ethylenoxids für den Block $E_n$,
$x(cat)$, als die Stoffmenge an eingesetztem Katalysator in mol,
$t_n$, als die Dosierzeit für das Ethylenoxid des Blocks $E_n$ in Stunden,
$x_n(EO)$, als die eindosierte Stoffmenge an Ethylenoxid für den Block $E_n$ in mol, und
n wie vorstehend

definiert sind, und
wobei alle Blöcke $A_m$ höchstens 40 Gew.% Oxyethyleneinheiten umfassen, und wobei der Gesamtgehalt an Oxyethyleneinheiten im Polyetherpolyol, bezogen auf die Gesamtmasse aller eindosierten Alkylenoxide, von 5 bis 45 Gew.-% beträgt.

Die Verwendung des Wortes *ein* im Zusammenhang mit abzählbaren Größen ist hierbei und im Folgenden nur dann als Zahlwort zu verstehen, wenn dies aus dem Zusammenhang hervorgeht (beispielsweise durch die Formulierung *"genau ein"*). Ansonsten umfassen Ausdrücke wie "ein Epoxid", "eine Starterverbindung" etc. immer auch solche Ausführungsformen, in denen zwei oder mehr Epoxide, zwei oder mehr Starterverbindungen etc. eingesetzt werden.

Das erfindungsgemäße Verfahren ermöglicht, dass bei Raumtemperatur feste Starterverbindungen mit Alkylenoxiden zu Polyetherpolyolen umgesetzt werden können, ohne dass hierbei der Einsatz von Lösungsmitteln, Costartern oder anderen Suspensionshilfen erforderlich ist. Dies ist sowohl unter wirtschaftlichen und prozesstechnischen Gesichtspunkten von Vorteil, da hierdurch zusätzliche Prozess- und Aufarbeitungsschritte entfallen, sowie allgemein erstrebenswert für die Entwicklung von nachhaltigen und ressourcenschonenden Prozessen. Das erfindungsgemäße Verfahren ermöglicht es, dass ohne den Einsatz von Lösungsmitteln im Ergebnis klare und homogene Alkylenoxiddditionsprodukte auf Basis der bei Raumtemperatur festen Starterverbindungen erhalten werden, welche als Komponenten von Polyurethanwerkstoffen mit Polyisocyanaten vielfältig eingesetzt werden können.

Unter Starterverbindungen im Sinne der Erfindung werden Verbindungen verstanden, die mindestens ein Zerewitinoff-aktives Wasserstoffatom aufweisen. An N, O oder S gebundener Wasserstoff wird als Zerewitinoff-aktiver Wasserstoff (oder als "aktiver Wasserstoff") bezeichnet, wenn er nach einem von Zerewitinoff aufgefundenen Verfahren durch Umsetzung mit Methylmagnesiumiodid Methan liefert. Typische Beispiele für Verbindungen mit Zerewitinoffaktivem Wasserstoff sind Verbindungen, die Carboxyl-, Hydroxyl-, Amino-, Imino- oder Thiol-Gruppen als funktionelle Gruppen enthalten. Unter bei Raumtemperatur festen Starterverbindungen werden solche Starterverbindungen verstanden, deren Schmelzpunkte bei 40 °C oder höher liegen und unter flüssigen Starterverbindungen solche mit Schmelzpunkten kleiner als 40 °C.

Nachfolgend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

Bei den Starterverbindungen handelt es sich erfindungsgemäß bevorzugt um Starterverbindungen, welche bei $\leq$ 40 °C in fester Form vorliegen. Beispiele hierfür sind Mono-, Oligo- und Polysaccharide, Pentaerythrit, Dipentaerythrit, Tripentaerythrit, Trimethylolethan, Trimethylolpropan, Sorbit, 1,12-Dodecandiol, 1,2-Decandiol, 1,10-Decandiol, cyclische Polyole (wie beispielsweise Inosit), aromatische Hydroxyverbindungen (wie beispielsweise Phenol, Hydrochinon, Brenzcatechin, Resorcin, 1,3,5-Trihydroxybenzol, Bisphenol-A oder Bisphenol-F), methylolgruppenhaltige Kondensate aus Formaldehyd und Phenol oder Melamin oder Harnstoff, Mannichbasen, hochfunktionelle Starterverbindungen auf Basis hydrierter Stärkehydrolyseprodukte, Polyamine (wie beispielsweise Verbindungen auf Basis hochfunktioneller mehrkerniger Anilin / Formaldehyd - Kondensationsprodukte ("polymeres MDA") und Isomere bzw. Isomerengemische des Toluylendiamins (insbesondere 2,4-TDA, 2,6-TDA, 2,3-TDA, 3,4-TDA). Es können auch Verbindungen mit Carbonsäuregruppen (wie beispielsweise Malonsäure, Glutarsäure oder Adipinsäure) oder Verbindungen mit Hydroxy- und Carbonsäurefunktionen (wie beispielsweise die Isomere der Hydroxybenzoesäure, die Isomere der Hydroxymethylbenzoesäure, die Isomere der Dihydroxybenzoesäure, die Isomere der Trihydroxybenzoesäure, Mandelsäure, Äpfelsäure, Citronensäure, Weinsäure und Schleimsäure) verwendet werden. Bevorzugt werden als Starterverbindungen Pentaerythrit, Saccharose, Trimethylolpropan und / oder Sorbit eingesetzt, besonders bevorzugt wird Sorbit eingesetzt.

Die erfindungsgemäß einzusetzenden Starterverbindungen können auch als Gemische eingesetzt werden. Gemeinsam mit den festen Starterverbindungen können erfindungsgemäß in kleinen Mengen, bis zu 30 Gew.-%, bezogen auf die Gesamtmasse aller Starterverbindungen, auch solche mit Schmelzpunkten niedriger als 40 °C eingesetzt werden. Bei diesen Starterverbindungen handelt es sich beispielsweise um Methanol, Ethanol, 1-Propanol, 2-Propanol und bei Raumtemperatur flüssige höhere aliphatische Monole, 1,2-Propylenglykol, 1,3-Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, Glycerin und Triethanolamin sowie Wasser. Erfindungsgemäß weisen mindestens 70 Gew.-% der eingesetzten Starterverbindungen einen Schmelzpunkt von ≥ 40 °C auf, wobei bevorzugt ausschließlich eine oder mehrere Starterverbindungen mit einem Schmelzpunkt von ≥ 40 °C eingesetzt werden.

Um die Handhabung der erfindungsgemäß zu verwendenden bei Raumtemperatur festen Starterverbindungen zu erleichtern (beispielsweise für den Deprotonierungsschritt bei der Umsetzung mit einem basischen Katalysator), können die Starterverbindungen in Form einer wässrigen Lösung eingesetzt werden und die Reaktionsmischung vor Beginn der eigentlichen Alkylenoxidadditionsreaktion bzw. nach Unterbrechung einer bereits laufenden Alkylenoxidadditionsreaktion, beispielsweise durch einen Strippschritt, entwässert werden. In einer bevorzugten Ausführungsform des erfindungsgemäßen Verfahrens werden bei der Umsetzung der Starterverbindung mit Ethylenoxid sowie der wenigstens einen weiteren Alkylenoxidverbindung keine Lösungsmittel verwendet.

Ein Verfahren ohne die Verwendung von Lösungsmitteln ist im Sinne der vorliegenden Erfindung derart zu verstehen, dass hierbei unter den üblichen Reaktionsbedingungen keine zusätzliche Zugabe von gegenüber Alkylenoxiden unreaktiven Lösungsmitteln und/oder Wasser erfolgt. Ein lösungsmittelfreies Verfahren im Sinne der vorliegenden Erfindung umfasst daher auch den Einsatz von Starterverbindungen und Alkylenoxiden, welche aufgrund ihres Herstellungsverfahrens noch Spuren von Lösungsmitteln (≤ 5 Gew.-%), insbesondere bis zu 2 Gew.-% Wasser, enthalten, sofern diese nicht zusätzlich beigegeben werden.

Für das erfindungsgemäße Verfahren werden als Alkylenoxide Ethylenoxid und Propylenoxid eingesetzt.

Die nach dem erfindungsgemäßen Verfahren hergestellten Polyetherpolyole weisen eine Blockstruktur auf mit mindestens einem Block $E_n$ und mindestens einem Block Am. Der oder die nach dem erfindungsgemäßen Verfahren zu dosierenden an Oxyethyleneinheiten reichen Blöcke $E_n$ mit einem Gehalt an Oxyethyleneinheiten von mindestens 75 Gew.-% können als innere Blöcke oder als Endblock dosiert werden. Bevorzugt enthalten die nach dem erfindungsgemäßen Verfahren hergestellten Polyetherpolyole einen Block $E_n$ als Endblock, da Produkte mit an Oxyethyleneinheiten reichen Endblöcken durch einen erhöhten Anteil an primären Endgruppen gekennzeichnet sind, welche den Polyetherpolyolen eine erhöhte Reaktivität gegenüber Isocyanaten verleihen. Vorzugsweise enthält mindestens ein Block $E_n$ wenigstens 90 Gew.-% Oxyethyleneinheiten, besonders bevorzugt enthält mindestens ein Block $E_n$ 100 Gew.-% Oxyethyleneinheiten.

In einer bevorzugten Ausführungsform der Erfindung bestehen die nach dem erfindungsgemäßen Verfahren hergestellten Polyetherpolyole neben den auf die Starter zurückgehenden Struktureinheiten ausschließlich aus den Blöcken $E_n$ und $A_m$. Alle Blöcke $A_m$ umfassen höchstens 40 Gew.% Oxyethyleneinheiten, bevorzugt höchstens 25 Gew.% Oxyethyleneinheiten.

Der Gesamtgehalt an Oxyethyleneinheiten in den nach dem erfindungsgemäßen Verfahren hergestellten Polyetherpolyolen beträgt, bezogen auf die Gesamtmasse aller eindosierten Alkylenoxide, von 5 bis 45 Gew.-%. Bevorzugt enthält das nach dem erfindungsgemäßen Verfahren hergestellte Polyetherpolyol bis zu 30 Gew.-% Oxyethyleneinheiten bezogen auf die Gesamtmasse aller eindosierten Alkylenoxide.

Die nach dem erfindungsgemäßen Verfahren hergestellten Polyetherpolyole sind also Blockcopolymere, aufgebaut aus an Oxyethyleneinheiten reichen Blöcken $E_n$ und aus an Oxyethyleneinheiten armen Blöcken Am. In einer bevorzugten Ausführungsweise wird bei der Herstellung dieser Blockcopolymere neben Ethylenoxid ausschließlich Propylenoxid als Alkylenoxid verwendet. Die an Oxyethyleneinheiten armen Blöcke Am sind somit bevorzugt reich an Oxypropyleneinheiten.

Wechsel der Blockzusammensetzung können während der Epoxiddosierphase diskontinuierlich oder auch, innerhalb kurzer Zeiträume, kontinuierlich vollzogen werden.

Im erfindungsgemäßen Verfahren werden basische Katalysatoren wie beispielsweise Alkalimetallhydride, Alkalimetallcarboxylate (beispielsweise von monofunktionellen Carbonsäuren), Alkalimetallhydroxide, Alkalimetallalkoxide (beispielsweise von monofunktionellen Alkoholen) oder Amine eingesetzt. Eine Übersicht über für das erfindungsgemäße Verfahren geeignete Amine ist von M. Ionescu et al. in "Advances in Urethanes Science and Technology", 1998, 14, S. 151-218 gegeben worden. Beispielsweise können N,N-Dimethylbenzylamin, Dimethylaminopropanol, N-Methyldiethanolamin, Trimethylamin, Triethylamin, N,N-Dimethylcyclohexylamin, N-Methylpyrrolidin, N,N,N',N'-Tetramethylethylendiamin, Diazabicyclo[2,2,2]-octan, 1,4-Dimethylpiperazin, N-Methylmorpholin, unsubstituiertes Imidazol und / oder alkylsubstituierte Imidazolderivate eingesetzt werden. Besonders bevorzugt werden im erfindungsgemäßen Verfahren als basische Katalysatoren Alkalimetallhydroxide (wie beispielsweise Natriumhydroxid, Kaliumhydroxid oder Cäsiumhydroxid), Alkalimetallalkoholate mono- oder mehrfunktioneller Alkohole, Imidazol oder alkylsubstituierte Imidazolderivate (wie beispielsweise N-Methylimidazol) eingesetzt. Ganz besonders bevorzugt wird im erfindungsgemäßen Verfahren Kaliumhydroxid eingesetzt.

Die Alkalimetallhydroxide können den bei Raumtemperatur festen Starterverbindungen als Feststoff oder als hochkonzentrierte wässrige Lösungen zugeführt werden. Durch einen der Alkylenoxiddosierphase vorgeschalteten Strippschritt können Lösungswasser und das Wasser, welches durch die Reaktion der Alkalimetallhydroxide mit den Zerewitinoff-aktiven Wasserstoffatomen der Starterverbindung entsteht, abgetrennt werden. Werden bei Alkalimetallhydroxidkatalyse wässrige Lösungen von bei Raumtemperatur festen Starterverbindungen eingesetzt, wird zweckmäßigerweise nur ein Strippschritt durchgeführt, also beispielsweise vor Beginn der eigentlichen Alkylenoxidadditionsphase oder, weniger bevorzugt, nach Unterbrechung einer bereits laufenden Alkylenoxidadditionsreaktion.

Bisweilen erweist es sich als vorteilhaft die für die Dosierung des einen oder mehrerer Blöcke $E_n$ nach Ungleichung (1) erforderliche Stoffmenge an eingesetztem Katalysator erst nach Dosierung eines ersten Blocks Am einzustellen. Hierbei eingetragenes Wasser und / oder durch die Reaktion der Alkalimetallhydroxide mit den Zerewitinoff-aktiven Wasserstoffen der Starterverbindung entstehendes Wasser wird vorzugsweise vor Wiederaufnahme der Alkylenoxidaddition abgetrennt.

Die basischen Katalysatoren werden im Allgemeinen in auf die Endproduktmenge bezogenen Mengen von 0,004 bis 1,0 Gew.-%, bevorzugt 0,02 bis 1,0 Gew.-%, eingesetzt, wobei während der Dosierung von dem oder den Blöcken mit einem Gehalt an Oxyethyleneinheiten von größer oder gleich 75 Gew.-% die in Ungleichung (1) formulierte Bedingung zu erfüllen ist.

Vorzugsweise erfolgt die Umsetzung der wenigstens einen Starterverbindung mit Ethylenoxid sowie wenigstens einer weiteren Alkylenoxidverbindung nach dem erfindungsgemäßen Verfahren bei einer Temperatur von 70 bis 170 °C, besonders bevorzugt bei einer Temperatur von 100 bis 160 °C. Die Temperatur kann während der Alkylenoxiddosierphase innerhalb der vorstehend beschriebenen Grenzen variiert werden, wobei während der Dosierung von dem oder den Blöcken $E_n$ im zeitlichen Mittel eine solche Reaktionstemperatur $T_n$ einzuhalten ist, dass die in Ungleichung (1) formulierte Bedingung erfüllt ist.

Um eine optimale Abstimmung zwischen hohem Alkylenoxidumsatz und geringer Nebenproduktbildung bei Verwendung empfindlicher Starterverbindungen (wie beispielsweise Saccharose) zu erreichen, kann zunächst bei niedrigen Reaktionstemperaturen (beispielsweise bei 70 bis 110 °C) alkoxyliert werden, und erst bei hinreichendem Starterumsatz (beispielsweise sobald mindestens 50 Gew.-% der eingesetzten Starterverbindungen an mindestens einem Zerewitinoff aktiven Wasserstoffatom mit Alkylenoxid reagiert haben) zu höheren Reaktionstemperaturen (beispielsweise auf 110 bis 130 °C) übergegangen werden. Nachreaktionen können ebenfalls bei höheren Temperaturen (d.h. nach Anhebung der Temperatur auf 100 bis 170 °C, bevorzugt 100 bis 150 °C) durchgeführt werden. Werden langkettige Polyetherpolyole, d. h. Polyetherpolyole mit Äquivalentmolmassen von größer 500 Da hergestellt, so wird empfohlen bei Erreichen von hohen Äquivalentmolmassen und bei Dosierung propylenoxidhaltiger Blöcke, beispielsweise bei 500 Da oder höheren Äquivalentmolmassen, die Reaktionstemperatur auf Werte von 120 °C, bevorzugt 110 °C oder niedriger, zu beschränken, um Nebenreaktionen des Propylenoxids, insbesondere seine Umlagerung in Allylalkohol auf ein tolerierbares Maß zu reduzieren. Das Ausmaß dieser Nebenreaktion nimmt naturgemäß mit dem Gehalt an Propylenoxid im dosierten Alkylenoxidgemisch zu, daher wird die Beschränkung der Reaktionstemperatur dann empfohlen, wenn der Propylenoxidgehalt im dosierten Alkylenoxidgemisch Werte von 30 Gew.-% überschreitet, insbesondere wenn er Werte von 50 Gew.-% oder gar 70 Gew.-% überschreitet. Die Dosierung hoch ethylenoxidhaltiger Blöcke oder von Blöcken aus reinem Ethylenoxid sowie Nachreaktionen können wiederum bei höheren Temperaturen (d.h. nach Anhebung der Temperatur auf 100 bis 170 °C, bevorzugt 100 bis 150 °C) durchgeführt werden. Die Temperatur der exothermen Alkylenoxidadditionsreaktion muss gegebenenfalls durch Kühlung auf dem gewünschten Niveau gehalten werden. Gemäß dem Stand der Technik zur Auslegung von Polymerisationsreaktoren für exotherme Reaktionen (z. B. Ullmann's Encyclopedia of Industrial Chemistry, Band B4, Seite 167ff, 5. Ausgabe, 1992) erfolgt eine solche Kühlung im Allgemeinen über die Reaktorwand (z. B. Doppelmantel, Halbrohrschlange) sowie mittels weiterer intern im Reaktor und/oder extern im Umpumpkreislauf angeordneter Wärmetauscherflächen, z.B. an Kühlschlangen, Kühlkerzen, Platten-, Rohrbündel- oder Mischerwärmetauschern. Diese sollten vorteilhafterweise so ausgelegt sein, dass auch zu Beginn der Dosierphase, d. h. bei kleinem Füllstand, und in Gegenwart eines heterogenen Reaktorinhalts (beispielsweise bei Vorliegen von Feststoffdispersionen) effektiv gekühlt werden kann.

Die berechneten OH-Zahlen der nach dem erfindungsgemäßen Verfahren erhältlichen Polyetherpolyole weisen bevorzugt Werte von 28 mg KOH/g bis 500 mg KOH/g, besonders bevorzugt von 50 mg KOH/g bis 400 mg KOH/g, ganz besonders bevorzugt von 80 mg KOH/g bis 350 mg KOH/g, auf.

Die OH-Zahl steht in der in Gleichung (2) wiedergegebenen Beziehung zur Äquivalentmolmasse. Unter Äquivalentmolmasse ist die durch die Zahl der aktiven Wasserstoffatome (Funktionalität) geteilte zahlenmittlere Gesamtmolmasse des aktive Wasserstoffatome enthaltenden Materials zu verstehen.

$$\text{OH-Zahl [mg KOH/g]} = 56100 \text{ [mg KOH / mol]} / \text{Äquivalentmolmasse [g / mol]} \qquad (2)$$

Der Reaktionsfortschritt bei der Umsetzung der wenigstens einen Starterverbindung mit Ethylenoxid sowie wenigstens einer weiteren Alkylenoxidverbindung nach dem erfindungsgemäßen Verfahren geht einher mit einer Abnahme der OH-Zahl, gemäß der in Gleichung (2) gegebenen Beziehung.

Es hat sich herausgestellt, dass trotz Erfüllung der in Ungleichung (1) formulierten Bedingung bisweilen Polyetherpolyole mit verhältnismäßig breiten Molmassenverteilungen erhalten werden. Polyetherpolyole mit besonders engen Molmassenverteilungen, d. h. niedrigen Polydispersitäten (PD), werden erhalten, wenn während der Umsetzung der wenigstens einen Starterverbindung mit Ethylenoxid sowie wenigstens einer weiteren Alkylenoxidverbindung folgende Ungleichung (3) gilt:

$$T_a * x(cat)/m_a < 12{,}5$$

$$\text{in } [°C * \text{mol/kg}] \qquad (3)$$

mit

$T_a$ als mittlere Reaktionstemperatur in °C während der Umsetzung der wenigstens einen Starterverbindung,
x(cat) als die Stoffmenge an eingesetztem Katalysator in mol und
$m_a$ als Masse des Ansatzes in kg nach erfolgter Umsetzung der wenigstens einen Starterverbindung.

Als Polydispersität (PD) ist die Molekulargewichtsverteilung $M_w/M_n$ definiert, wobei $M_w$ für die gewichtsgemittelte Molmasse und $M_n$ für die zahlengemittelte Molmasse stehen.

In einer bevorzugten Ausführungsform der Erfindung gilt Ungleichung (3) ab einer OH-Zahl von 1000 mg KOH / g oder weniger, besonders bevorzugt ab 850 mg KOH / g oder weniger. Die untere Grenze des OH-Zahl-Bereiches für den Ungleichung (3) gilt, ist vorgegeben durch die OH-Zahl des Polyetherpolyols nach erfolgter Umsetzung der wenigstens einen Starterverbindung. Bevorzugt gilt Ungleichung (3) bis zu einer OH-Zahl von 215 mg KOH / g, wobei $m_a$ die Masse des Ansatzes in kg bei Passieren der OH-Zahl 215 mg KOH / g bezeichnet.

Generell ist in allen Reaktionsphasen durch Auslegung und Einsatz handelsüblicher Rührorgane für eine gute Durchmischung des Reaktorinhaltes zu sorgen, wobei hier insbesondere ein- oder mehrstufig angeordnete Rührer oder großflächig über die Füllhöhe wirkende Rührertypen geeignet sind (siehe z.B. Handbuch Apparate; Vulkan-Verlag Essen, 1. Aufl. (1990), S.188 - 208). Besonders technisch relevant ist hierbei eine im Mittel über den gesamten Reaktorinhalt eingetragene volumenspezifische Mischleistung, die im Allgemeinen im Bereich von 0,2 W/1 bis 5 W/1, bezogen auf das Reaktorvolumen, liegt, mit entsprechend höheren volumenspezifischen lokalen Leistungseinträgen im Bereich der Rührorgane selbst und gegebenenfalls bei niedrigeren Füllständen. Um eine optimale Rührwirkung zu erzielen, können im Reaktor gemäß allgemeinem Stand der Technik Kombinationen aus Stromstörern (beispielsweise Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Leistungseintrag zu gewährleisten. Beispielsweise kann es vorteilhaft sein, feststoffhaltige Dispersionen, die zu Reaktionsbeginn bei der (Mit-)Verwendung von Saccharose vorliegen können, oder viskose Schmelzen von bei Raumtemperatur festen Startern, besonders intensiv zu durchmischen. Außerdem ist beim Einsatz fester Starter durch die Wahl des Rühraggregates sicherzustellen, dass eine ausreichende Dispergierung des Feststoffes im Reaktionsgemisch gewährleistet ist. Bevorzugt werden hier bodengängige Rührstufen sowie besonders zur Suspendierung geeignete Rührorgane eingesetzt. Ferner sollte die Rührergeometrie zur Minderung des Aufschäumens von Reaktionsprodukten beitragen. Das Aufschäumen von Reaktionsgemischen kann beispielsweise nach Ende der Dosier- und Nachreaktionsphase beobachtet werden, wenn Restalkylenoxide zusätzlich im Vakuum bei absoluten Drücken im Bereich von 1 mbar bis 500 mbar entfernt werden. Für solche Fälle haben sich Rührorgane als geeignet herausgestellt, die eine kontinuierliche Durchmischung der Flüssigkeitsoberfläche erzielen. Je nach Anforderung weist die Rührwelle ein Bodenlager und ggf. weitere Stützlager im Behälter auf. Der Antrieb der Rührerwelle kann dabei von oben oder unten erfolgen (mit zentrischer oder exzentrischer Anordnung der Welle).

Alternativ ist es auch möglich, die notwendige Durchmischung ausschließlich mittels eines über einen Wärmetauscher geführten Umpumpkreislaufs zu erzielen oder diesen zusätzlich zum Rühraggregat als weitere Mischkomponente zu betreiben, wobei der Reaktorinhalt nach Bedarf (typischerweise 1 bis 50 mal pro Stunde) umgepumpt wird. Die mittels Umpumpung, beispielsweise über einen außenliegenden Wärmetauscher über diesen oder bei Rückführung in den Reaktor über eine Düse oder Injektor, eingetragene spezifische Mischenergie beläuft sich ebenfalls auf Werte von im Mittel 0,2 bis 5 W/L, wobei diese auf das im Reaktor und den Umpumpkreislauf am Ende der Reaktionsphase befindliche Flüssigkeitsvolumen bezogen ist.

Die Alkylenoxide können dem Reaktor auf unterschiedliche Weise zugeführt werden: Möglich ist eine Dosierung in die Gasphase oder direkt in die Flüssigphase, beispielsweise über ein Tauchrohr oder einen in der Nähe des Reaktorbodens in einer gut durchmischten Zone befindlichen Verteilerring. Die kontinuierliche Dosierung des wenigstens einen Alkylenoxids erfolgt so, dass die sicherheitstechnischen Druckgrenzen nicht überschritten werden. Diese richten sich naturgemäß nach den im Einzelfall vorliegenden apparativen Gegebenheiten, wobei der Prozess im Allgemeinen in einem Druckbereich von 1 mbar bis 10 bar, besonders bevorzugt von 1 mbar bis 4 bar ausgeführt wird. Insbesondere bei der Dosierung von ethylenoxidhaltigen Alkylenoxidgemischen oder reinem Ethylenoxid ist vorteilhafterweise darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden. Bei Dosierung in die Flüssigphase sollten die Dosieraggregate selbstleerend ausgelegt sein, beispielsweise durch Anbringen der Dosierbohrungen an der Unterseite des Verteilerrings. Generell sollte durch apparative Maßnahmen, beispielsweise durch die Montage von Rückschlagklappen, ein Rückströmen von Reaktionsmedium in die Dosieraggregate und Eduktvorlagen verhindert werden. Wird ein Alkylenoxidgemisch dosiert, können die jeweiligen Alkylenoxide dem Reaktor separat oder als Mischung zugeführt werden. Eine Vorvermischung der Alkylenoxide untereinander kann beispielsweise durch ein in der gemeinsamen Dosierstrecke befindliches Mischaggregat erreicht werden ("inline-blending"). Es hat sich auch bewährt, die Alkylenoxide pumpendruckseitig in einen beispielsweise über einen oder mehrere Wärmetauscher geführten Umpumpkreislauf einzeln oder vorgemischt zu dosieren. Für die gute Durchmischung mit dem Reaktionsmedium ist es dann von Vorteil ein hochscherendes Mischaggregat in den Alkylenoxid- / Reaktionsmediumstrom zu integrieren.

Generell sind die unterschiedlichsten Reaktortypen für die Durchführung des erfindungsgemäßen Verfahrens geeignet. Im Allgemeinen werden zylinderförmige Behälter eingesetzt, welche ein Höhen- zu Durchmesserverhältnis von 1:1 bis 10:1 besitzen. Als Reaktorböden kommen beispielsweise Kugel-, Klöpper-, Flach- oder Konusböden in Frage.

Nach Ende der Epoxiddosierung oder bei einem Wechsel der Zusammensetzung des dosierten Alkylenoxidgemisches können sich Nachreaktionsphasen anschließen, in denen restliches Alkylenoxid abreagiert. Das Ende einer solchen Nachreaktionsphase ist erreicht, wenn kein weiterer Druckabfall im Reaktionskessel mehr feststellbar ist. Spuren unreagierter Alkylenoxide können nach der (Nach-)Reaktionsphase gegebenenfalls im Vakuum bei einem absoluten Druck von 1 mbar bis 500 mbar oder durch Strippen quantitativ entfernt werden. Durch Strippen werden flüchtige Bestandteile, wie beispielsweise (Rest-)Alkylenoxide, unter Einleiten von Inertgasen oder Wasserdampf in die Flüssigphase bei gleichzeitig angelegtem Vakuum, beispielsweise durch Durchleiten von Inertgas bei einem Absolutdruck von 5 mbar bis 500 mbar. entfernt. Das Entfernen flüchtiger Bestandteile, wie beispielsweise nicht umgesetzter Alkylenoxide, entweder im Vakuum oder durch Strippen, erfolgt bei Temperaturen von 20 °C bis 200 °C, bevorzugt bei 50 °C bis 160 °C und vorzugsweise unter Rühren. Die Strippvorgänge können auch in sog. Strippkolonnen durchgeführt werden, in denen dem Produktstrom ein Inertgas- oder Wasserdampfstrom entgegengeleitet wird. Bevorzugt wird das Strippen mit Inertgasen in Abwesenheit von Wasserdampf durchgeführt.

Nach Erreichen von Druckkonstanz bzw. nach Entfernen flüchtiger Bestandteile im Vakuum und/oder Strippen kann das Produkt optional Aufarbeitungsschritten unterzogen werden, um etwaige Katalysatorspuren zu entfernen. Im Falle von mit Aminen katalysierten Alkylenoxidadditionsreaktionen sind solche Nachbehandlungsschritte im Allgemeinen nicht erforderlich. Die optionale Entfernung des Katalysators aus dem Rohprodukt kann auf verschiedene Weise erfolgen: Beispielsweise kann der basische Katalysator mit verdünnten Mineralsäuren wie Schwefelsäure oder Phosphorsäure neutralisiert werden. Die bei der Neutralisation entstehenden Salze werden abgetrennt, beispielsweise durch Filtration. Ausnahmen bilden die in EP-A 2028211, WO-A 2009/152954, WO-A 2011/039145 und WO-A 2009/106244 beschriebenen Polyetherpolyolherstellungsverfahren, die Aufarbeitungsverfahren ohne Salzabtrennungsschritte zum Inhalt haben. Alternativ kann die Neutralisation mit Hydroxycarbonsäuren (wie beispielsweise Milchsäure, wie in WO-A 98/20061 und US-A 2004/167316 beschrieben) erfolgen. Ebenso geeignet zur Neutralisation sind Carbonsäuren wie beispielsweise Ameisensäure, wie in US 4,521,548 beschrieben. Die durch die Neutralisation mit Carbonsäuren (wie beispielsweise Hydroxycarbonsäuren oder Ameisensäure) entstehenden Metallcarboxylate sind in dem Polyetherpolyol klar löslich, so dass die Abtrennung der Salze hier ebenfalls entfallen kann. Ebenfalls möglich zur Entfernung des Katalysators ist der Einsatz von sauren Kationenaustauschern, wie beispielsweise in DE-A 100 24 313 beschrieben. Des Weiteren können die Katalysatoren mittels Adsorbentien, wie beispielsweise Schichtsilikaten (Bentonit, Attapulgit), Diatomeenerde oder auch mit Hilfe synthetischer Magnesiumsilikate (wie AMBOSOL® oder BriteSorb®) abgetrennt werden. Solche Aufreinigungsverfahren sind beschrieben in RO 118433, US 4,507,475, EP-A 0693513 und EP-A 1751213. Phasentrennverfahren, die gegebenenfalls durch hydrophobe Lösungsmittel unterstützt werden, sind prinzipiell ebenfalls möglich, jedoch kann die Wasserlöslichkeit des auf Basis der bei Raumtemperatur festen Starterverbindungen hergestellten Oxyethyleneinheiten enthaltenden Polyetherpolyols für die effektive Durchführung von Phasentrennverfahren zu hoch sein. Phasentrennverfahren sind beispielsweise beschrieben in WO-A 01/14456, JP-A 6-157743, WO-A 96/20972 und US-A 3,823,145.

Es hat sich als vorteilhaft erwiesen, Polyole ganz generell stets unter Inertgasatmosphäre zu handhaben. Dies gilt insbesondere für alkalische Polyetherpolyole, wie sie beispielsweise unter Alkalimetallhydroxidkatalyse vor Abtrennung des Katalysators anfallen oder für unter Aminkatalyse erhaltene Produkte. Auch für salzfreie, aufgearbeitete und stabilisierte Fertigprodukte oder über Doppelmetallcyanidkatalyse (DMC-Katalyse) hergestellte Polyetherpolyole werden Handhabung und Lagerung unter Sauerstoffausschluss empfohlen. Hierfür geeignete Inertgase sind beispielsweise Edelgase, Stickstoff oder Kohlendioxid, besonders geeignet sind Edelgase oder Stickstoff. Durch die Unterbindung von Sauerstoffzutritt lassen sich Produktverfärbungen weitestgehend vermeiden, dies gilt insbesondere bei erhöhten Temperaturen, die im Allgemeinen genutzt werden um durch Senkung der Produktviskosität die Handhabung der Polyetherpolyole zu erleichtern. Darüber hinaus entstehen unter Inertgasatmosphäre auch deutlich weniger Peroxidgruppen, die unter Spaltung der Polyetherketten zur Bildung weiterer niedermolekularer oxidativer Abbauprodukte wie beispielsweise Acetaldehyd, Methanol, Ameisensäure, Ameisensäureester, Aceton und Formaldehyd beitragen. Somit können der Gehalt an leichtflüchtigen organischen Verbindungen in den Polyetherpolyolen gesenkt und Geruchsbelästigungen, gesundheitliche Beeinträchtigungen sowie Qualitätsminderungen vermieden werden. Den nach dem erfindungsgemäßen Verfahren hergestellten Polyetherpolyolen können gegebenenfalls Alterungsschutzmittel wie z. B. Antioxidantien zugesetzt werden. Nach dem erfindungsgemäßen Verfahren lassen sich Polyetherpolyole herstellen, welche bei 20 °C insbesondere optisch klar und / oder homogen sind.

Die nach dem erfindungsgemäßen Verfahren hergestellten Polyetherpolyole können als Ausgangskomponenten für die Herstellung von massiven oder geschäumten Polyurethanwerkstoffen sowie von Polyurethanelastomeren eingesetzt werden. Die Polyurethanwerkstoffe und -elastomere können auch Isocyanurat-, Allophanat- und Biuretstruktureinheiten enthalten. Ebenfalls möglich ist die Herstellung sogenannter Isocyanat-Präpolymere, bei deren Herstellung mindestens ein (Poly-)Isocyanat und mindestens ein nach dem erfindungsgemäßen Verfahren erhältliches Polyetherpolyol eingesetzt werden, wobei das molare Verhältnis von Isocyanatgruppen zu Hydroxygruppen größer als 1 ist, so dass die resultierenden Präpolymere Isocyanatgruppen enthalten. Die Isocyanatgruppen der Präpolymere können in einem oder mehreren Schritten mit Zerewitinoff-aktive Wasserstoffatome enthaltenden Verbindungen umgesetzt werden zur Herstellung der eigentlichen Endprodukte, wie beispielsweise massiven oder geschäumten Polyurethanwerkstoffen oder Polyurethanelastomeren. In umgekehrter Weise ist es auch möglich (Poly-)Isocyanate und mindestens ein nach dem erfindungsgemäßen Verfahren erhältliches Polyetherpolyol so umzusetzen, dass das molare Verhältnis von Isocyanatgruppcn zu Hydroxygruppen kleiner als 1 ist und somit die resultierenden Vorpolymerisate Hydroxygruppen enthalten. Die Hydroxygruppen der Vorpolymerisate können in einem oder mehreren Schritten mit Isocyanatgruppenhaltigen Verbindungen umgesetzt werden zur Herstellung der eigentlichen Endprodukte wie massiven oder geschäumten Polyurethanwerkstoffen oder Polyurethanelastomeren.

Zur Herstellung von massiven oder geschäumten Polyurethanwerkstoffen sowic von Polyurethanelastomeren wird mindestens ein nach dem erfindungsgemäßen Verfahren erhältliches Polyetherpolyol gegebenenfalls mit weiteren isocyanatreaktiven Komponenten gemischt und mit organischen Polyisocyanaten, gegebenenfalls in Gegenwart von Treibmitteln, Katalysatoren und / oder anderer Zusatzstoffe wie z. B. Zellstabilisatoren, zur Reaktion gebracht.

**Beispiele**

**Eingesetzte Rohstoffe:**

**IRGANOX® 1076:**

Octadecyl-3-(3,5-di-*tert*-butyl-4-hydroxyphenyl)propionat (Ciba Specialty Chemicals (jetzt BASF))

**Mess- und Bestimmungsmethoden:**

**OH-Zahl und Viskosität**

Die Bestimmung der OH-Zahlen erfolgte gemäß der Vorschrift der DIN 53240. Die Viskositäten wurden mittels Rotationsviskosimeter (Physica MCR 51, Hersteller: Anton Paar) nach der Vorschrift der DIN 53019 ermittelt (Spindeltyp CC27, Scherratenbereich 16 - 128 $s^{-1}$).

**Molmassenverteilung**

Die Molmassenverteilungen wurden mittels Größenausschlusschromatographie (SEC) ermittelt. Verwendet wurde das Gerät Agilent 1100 Series der Fa. Agilent. Angegeben wird die Polydispersität (PD) für die Molekulargewichtsverteilung $M_w/M_n$, wobei $M_w$ für die gewichtsgemittelte Molmasse und $M_n$ für die zahlengemittelte Molmasse stehen. Weitere Angaben:

- Säulenkombination: 1 Vorsäule PSS, 5 µl, 8x50mm; 2 PSS SVD, 5 µl, 100 A°, 8x300mm; 2 PSS SVD, 5 µl, 1000 A°, 8x300mm, PSS ist der Hersteller der Säulen (Polymer Standard Service, Mainz)
- Auswertesoftware: WinGPC der Fa. PSS
- Lösungsmittel: THF (Merck LiChrosolv®)
- Flussrate: 1 mL / min
- Detektortyp: RI-Detektor (Brechungsindex), Shodex RI-74
- Verwendete Kalibrationsstandards: Kalibrierstandard der Fa. PSS auf Basis Polystyrol.

**Beispiel 1:**

In einen 2 L Laborautoklaven wurden unter Stickstoffatmosphäre 163,5 g einer wässrigen Sorbitlösung, enthaltend 70 Gew.-% Sorbit, und 10,797 g wässrige KOH-Lösung, enthaltend 44,78 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 130 °C und dosierte 875,5 g Propylenoxid bei einer Rührerdrehzahl von 800 U/min. über einen Zeitraum von 3,07 h in den Kopfraum des Autoklaven, man begann die Dosierung bei einem absoluten Druck von 0,05 bar. Nach Ende der Propylenoxiddosierung schloss sich eine Nachreaktionszeit von 1,43 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 218,9 g Ethylenoxid ("oxyethyleneinheitenreicher Block") über einen Zeitraum von 3,2 h ebenfalls bei 130 ° C in den Kopfraum des Autoklaven dosiert. Nach einer Nachreaktionszeit von 3,68 h wurde das Produkt im Vakuum (10 mbar) über einen Zeitraum von 30 min. ausgeheizt. Nach Abkühlen auf 80 °C wurden 120 mL destilliertes Wasser und 35,23 g einer 12,22 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,1093 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 182 mg KOH / g und einer Viskosität von 808 mPas (bei 25 °C) erhalten. Die Polydispersität betrug 1,09.

**Beispiel 2**

In einen 2 L Laborautoklaven wurden unter Stickstoffatmosphäre 163,0 g einer wässrigen Sorbitlösung, enthaltend 70 Gew.-% Sorbit, und 5,380 g wässrige KOH-Lösung, enthaltend 44,76 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 872,8 g Propylenoxid bei einer Rührerdrehzahl von 800 U/min. über einen Zeitraum von 3,07 h in den Kopfraum des Autoklaven, man begann die Dosierung bei einem absoluten Druck von 0,05 bar. Nach Ende der Propylenoxiddosierung schloss sich eine Nachreaktionszeit von 0,67 h an. Die zu diesem Zeitpunkt Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 218,2 g Ethylenoxid ("oxyethyleneinheitenreicher Block") über einen Zeitraum von 3,2 h ebenfalls bei 150 °C in den Kopfraum des Autoklaven dosiert. Nach einer Nachreaktionszeit von 3,11 h wurde das Produkt im Vakuum (10 mbar) über einen Zeitraum von 30 min. ausgeheizt. Nach Abkühlen auf 80 °C wurden 120 mL destilliertes Wasser und 17,40 g einer 12,22 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,1038 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 172 mg KOH / g und einer Viskosität von 843 mPas (bei 25 °C) erhalten. Die Polydispersität betrug 1,03.

**Beispiel 3**

In einen 2 L Laborautoklaven wurden unter Stickstoffatmosphäre 163,1 g einer wässrigen Sorbitlösung, enthaltend 70 Gew.-% Sorbit, und 10,850 g wässrige KOH-Lösung, enthaltend 44,76 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 120 g Propylenoxid bei einer Rührerdrehzahl von 800 U/min. in den Autoklaven, man begann die Dosierung bei einem absoluten Druck von 0,05 bar. Der Inhalt des Autoklaven wurde sodann auf 120 °C abgekühlt. Bei 120 °C wurden weitere 749 g Propylenoxid zugegeben. Die gesamte Propylenoxidmenge wurde über einen Zeitraum von 3,88 h zugegeben. Nach Ende der Propylenoxiddosierung wurde wieder auf 150 °C erwärmt und es schloss sich eine Nachreaktionszeit von 1 h an. Die zu diesem Zeitpunkt Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 214 mg KOH / g. Der Reaktordruck wurde sodann mittels Stickstoff auf 2,75 bar eingestellt und es wurden 217,3 g Ethylenoxid ("oxyethyleneinheitenreicher Block") über einen Zeitraum von 3,15 h ebenfalls bei 150 °C in den Kopfraum des Autoklaven dosiert. Nach einer Nachreaktionszeit von 0,55 h wurde das Produkt im Vakuum (10 mbar) über einen Zeitraum von 30 min. ausgeheizt. Nach Abkühlen auf 80 °C wurden 120 mL destilliertes Wasser und 17,40 g einer 12,22 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,1038 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 171 mg KOH / g und einer Viskosität von 847 mPas (bei 25 °C) erhalten. Die Polydispersität betrug 1,03.

**Beispiel 4**

In einen 2 L Laborautoklaven wurden unter Stickstoffatmosphäre 163,7 g einer wässrigen Sorbitlösung, enthaltend 70 Gew.-% Sorbit, und 5,449 g wässrige KOH-Lösung, enthaltend 44,76 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 120 g Propylenoxid bei einer Rührerdrehzahl von 800 U/min. in den Autoklaven, man begann die Dosierung bei einem absoluten Druck von 0,05 bar. Der Inhalt des Autoklaven wurde sodann auf 120 °C abgekühlt. Bei 120 °C wurden weitere 753 g Propylenoxid zugegeben. Die gesamte Propylenoxidmenge wurde über einen Zeitraum von 5,6 h zugegeben. Nach Ende der Propylenoxiddosierung wurde wieder auf 150 °C erwärmt und es schloss sich eine Nachreaktionszeit von 2,17 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 219,2 g Ethylenoxid ("oxyethyleneinheitenreicher Block") über einen Zeitraum von 3,23 h ebenfalls bei 150 °C in den Kopfraum des Autoklaven dosiert. Nach einer Nachreaktionszeit von 1,05 h wurde das Produkt im Vakuum (10 mbar) über einen Zeitraum von 30 min. ausgeheizt. Nach Abkühlen auf 80 °C wurden 120 mL destilliertes Wasser und 17,58 g einer 12,22 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,100 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 171 mg KOH / g und einer Viskosität von 836 mPas (bei 25 °C) erhalten. Die Polydispersität betrug 1,03.

**Beispiel 5**

In einen 2 L Laborautoklaven wurden unter Stickstoffatmosphäre 162,9 g einer wässrigen Sorbitlösung, enthaltend 70 Gew.-% Sorbit, und 10,790 g wässrige KOH-Lösung, enthaltend 44,76 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 872,6 g Propylenoxid bei einer Rührerdrehzahl von 800 U/min. über einen Zeitraum von 2,38 h in den Kopfraum des Autoklaven, man begann die Dosierung bei einem absoluten Druck von 0,05 bar. Nach Ende der Propylenoxiddosierung schloss sich eine Nachreaktionszeit von 0,83 h an. Die zu diesem auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 218,1 g Ethylenoxid ("oxyethyleneinheitenreicher Block") über einen Zeitraum von 3,22 h ebenfalls bei 150 °C in den Kopfraum des Autoklaven dosiert. Nach einer Nachreaktionszeit von 0,67 h wurde das Produkt im Vakuum (10 mbar) über einen Zeitraum von 30 min. ausgeheizt. Nach Abkühlen auf 80 °C wurden 120 mL destilliertes Wasser und 35,22 g einer 12,22 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,0969 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 182 mg KOH / g und einer Viskosität von 773 mPas (bei 25 °C) erhalten. Die Polydispersität betrug 1,18.

**Beispiel 6**

In einen 2 L Laborautoklaven wurden unter Stickstoffatmosphäre 163,1 g einer wässrigen Sorbitlösung, enthaltend 70 Gew.-% Sorbit, und 22,022 g wässrige KOH-Lösung, enthaltend 44,76 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 873,4 g Propylenoxid bei einer Rührerdrehzahl von 800 U/min. über einen Zeitraum von 3,50 h in den Kopfraum des Autoklaven, man begann die Dosierung bei einem absoluten Druck von 0,05 bar. Nach Ende der Propylenoxiddosierung schloss sich eine Nachreaktionszeit von 0,58 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 212 mg KOH / g. Der Reaktordruck wurde sodann mittels Stickstoff auf 2,75 bar eingestellt und es wurden 218,4 g Ethylenoxid ("oxyethyleneinheitenreicher Block") über einen Zeitraum von 1,12 h ebenfalls bei 150 °C in den Kopfraum des Autoklaven dosiert. Nach einer Nachreaktionszeit von 0,17 h wurde das Produkt im Vakuum (10 mbar) über einen Zeitraum von 30 min. ausgeheizt. Nach Abkühlen auf 80 °C wurden 120 mL destilliertes Wasser und 70,50 g einer 12,22 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,0978 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 181 mg KOH / g und einer Viskosität von 773 mPas (bei 25 °C) erhalten. Die Polydispersität betrug 1,15.

**Beispiel 7**

In einen 2 L Laborautoklaven wurden unter Stickstoffatmosphäre 92,9 g einer wässrigen Sorbitlösung, enthaltend 70 Gew.-% Sorbit, und 2,985 g wässrige KOH-Lösung, enthaltend 44,59 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 908,3 g Propylenoxid bei einer Rührerdrehzahl von 800 U/min. über einen Zeitraum von 5,7 h in den Kopfraum des Autoklaven, man begann die Dosierung bei einem absoluten Druck von 0,05 bar. Nach Ende der Propylenoxiddosierung schloss sich eine Nachreaktionszeit von 3,0 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 123 mg KOH / g. Der Reaktordruck wurde sodann mittels Stickstoff auf 2,75 bar eingestellt und es wurden 227,0 g Ethylenoxid ("oxyethyleneinheitenreicher Block") über einen Zeitraum von 6,08 h ebenfalls bei 150 °C in den Kopfraum des Autoklaven dosiert. Nach einer Nachreaktionszeit von 0,87 h wurde das Produkt im Vakuum (10 mbar) über einen Zeitraum von 30 min. ausgeheizt. Nach Abkühlen auf 80 °C wurden 120 mL destilliertes Wasser und 10,356 g einer 11,74 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 0,6008 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 104 mg KOH / g und einer Viskosität von 656 mPas (bei 25 °C) erhalten. Die Polydispersität betrug 1,03.

**Beispiel 8**

In einen 2 L Laborautoklaven wurden unter Stickstoffatmosphäre 279,1 g einer wässrigen Sorbitlösung, enthaltend 70 Gew.-% Sorbit, und 8,552 g wässrige KOH-Lösung, enthaltend 46,59 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 804,4 g Propylenoxid bei einer Rührerdrehzahl von 800 U/min. über einen Zeitraum von 4,1 h in den Kopfraum des Autoklaven, man begann die Dosierung bei einem absoluten Druck von 0,05 bar. Nach Ende der Propylenoxiddosierung schloss sich eine Nachreaktionszeit von 1,2 h an. Der Reaktordruck wurde sodann mittels Stickstoff auf 2,75 bar eingestellt und es wurden 201,0 g Ethylenoxid ("oxyethyleneinheitenreicher Block") über einen Zeitraum von 1,83 h ebenfalls bei 150 °C in den Kopfraum des Autoklaven dosiert. Nach einer Nachreaktionszeit von 0,93 h wurde das Produkt im Vakuum (10 mbar) über einen Zeitraum von 30 min. ausgeheizt. Nach Abkühlen auf 80 °C wurden 120 mL destilliertes Wasser und 30,405 g einer 11,74 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 0,600 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares Produkt mit einer OH-Zahl von 290 mg KOH / g und einer Viskosität von 2470 mPas (bei 25 °C) erhalten. Die Polydispersität betrug 1,03.

**Beispiel 9 (Vergleich)**

In einen 2 L Laborautoklaven wurden unter Stickstoffatmosphäre 163,8 g einer wässrigen Sorbitlösung, enthaltend 70 Gew.-% Sorbit, und 10,948 g wässrige KOH-Lösung, enthaltend 44,78 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 120 °C und dosierte 877,1 g Propylenoxid bei einer Rührerdrehzahl von 800 U/min. über einen Zeitraum von 11,6 h in den Kopfraum des Autoklaven, man begann die Dosierung bei einem absoluten Druck von 0,05 bar. Nach Ende der Propylenoxiddosierung schloss sich eine Nachreaktionszeit von 1,0 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 219,3 g Ethylenoxid ("oxyethyleneinheitenreicher Block") über einen Zeitraum von 1,3 h ebenfalls bei 120 °C in den Kopfraum des Autoklaven dosiert. Nach einer Nachreaktionszeit von 1,0 h wurde das Produkt im Vakuum (10 mbar) über einen Zeitraum von 30 min. ausgeheizt. Nach Abkühlen auf 80 °C wurden 120 mL destilliertes Wasser und 36,156 g einer 12,13 gew.-%igen wässrigen Schwefelsäurelösung zugegeben und bei 80 °C über einen Zeitraum von 30 min. verrührt. Das entstandene Gemisch wurde in einen Glaskolben abgelassen und es würden 1,9725 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein trübes Produkt mit einer OH-Zahl von 162 mg KOH / g erhalten. Das Material erstarrte bei Raumtemperatur zu einer festen wachsartigen Substanz. Die Polydispersität betrug 1,10.

**Beispiel 10 (Vergleich)**

In einen 2 L Laborautoklaven wurden unter Stickstoffatmosphäre 162,8 g einer wässrigen Sorbitlösung, enthaltend 70 Gew.-% Sorbit, und 10,775 g wässrige KOH-Lösung, enthaltend 44,78 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 120 °C und dosierte 869,1 g Propylenoxid bei einer Rührerdrehzahl von 800 U/min. über einen Zeitraum von 7,9 h in den Kopfraum des Autoklaven, man begann die Dosierung bei einem absoluten Druck von 0,05 bar. Nach Ende der Propylenoxiddosierung schloss sich eine Nachreaktionszeit von 3,85 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 217,3 g Ethylenoxid ("oxyethyleneinheitenreicher Block") über einen Zeitraum von 0,63 h ebenfalls bei 120 °C in den Kopfraum des Autoklaven dosiert. Nach einer Nachreaktionszeit von 1,18 h wurde das Produkt im Vakuum (10 mbar) über einen Zeitraum von 30 min. ausgeheizt. Nach Abkühlen auf 80 °C wurden 120 mL destilliertes Wasser und 34,824 g einer 12,13 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,3614 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein zunächst klares aber rasch eintrübendes Produkt mit einer OH-Zahl von 164 mg KOH / g und einer Viskosität von 1700 mPas (bei 25 °C) erhalten. Die Polydispersität betrug 1,04.

**Beispiel 11 (Vergleich)**

In einen 2 L Laborautoklaven wurden unter Stickstoffatmosphäre 162,8 g einer wässrigen Sorbitlösung, enthaltend 70 Gew.-% Sorbit, und 10,829 g wässrige KOH-Lösung, enthaltend 44,78 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 130 °C und dosierte 872,0 g Propylenoxid bei einer Rührerdrehzahl von 800 U/min. über einen Zeitraum von 5,2 h in den Kopfraum des Autoklaven, man begann die Dosierung bei einem absoluten Druck von 0,05 bar. Nach Ende der Propylenoxiddosierung schloss sich eine Nachreaktionszeit von 2,37 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 218,0 g Ethylenoxid ("oxyethyleneinheitenreicher Block") über einen Zeitraum von 0,8 h ebenfalls bei 130 °C in den Kopfraum des Autoklaven dosiert. Nach einer Nachreaktionszeit von 0,58 h wurde das Produkt im Vakuum (10 mbar) über einen Zeitraum von 30 min. ausgeheizt. Nach Abkühlen auf 80 °C wurden 120 mL destilliertes Wasser und 35,635 g einer 12,13 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,0037 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein zunächst klares aber rasch eintrübendes und später wachsartig erstarrendes Produkt mit einer OH-Zahl von 167 mg KOH / g erhalten. Die Polydispersität betrug 1,03.

**Beispiel 12 (Vergleich)**

In einen 2 L Laborautoklaven wurden unter Stickstoffatmosphäre 162,5 g einer wässrigen Sorbitlösung, enthaltend 70 Gew.-% Sorbit, und 10,762 g wässrige KOH-Lösung, enthaltend 44,78 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 mL Stickstoff pro Minute über einen Zeitraum von 3 h entwässert. Hierbei stellte sich gegen Ende der Entwässerungszeit ein Druck von 100 - 120 mbar ein. Man erwärmte auf 150 °C und dosierte 869,1 g Propylenoxid bei einer Rührerdrehzahl von 800 U/min. über einen Zeitraum von 3,18 h in den Kopfraum des Autoklaven, man begann die Dosierung bei einem absoluten Druck von 0,05 bar. Nach Ende der Propylenoxiddosierung schloss sich eine Nachreaktionszeit von 0,63 h an. Die zu diesem Zeitpunkt auf Basis der OH-Zahl der Starterverbindung, sowie der Stoffmengen an Starterverbindung, Katalysator und Propylenoxid, berechnete OH-Zahl des Reaktorinhaltes betrug 213 mg KOH / g. Der Reaktordruck wurde sodann mittels Stickstoff auf 2,75 bar eingestellt und es wurden 217,3 g Ethylenoxid ("oxyethyleneinheitenreicher Block") über einen Zeitraum von 0,92 h ebenfalls bei 150 °C in den Kopfraum des Autoklaven dosiert. Nach einer Nachreaktionszeit von 0,55 h wurde das Produkt im Vakuum (10 mbar) über einen Zeitraum von 30 min. ausgeheizt. Nach Abkühlen auf 80 °C wurden 120 mL destilliertes Wasser und 34,670 g einer 12,13 gew.-%igen wässrigen Schwefelsäurelösung zugegeben und bei 80 °C über einen Zeitraum von 30 min. verrührt. Das entstandene Gemisch wurde in einen Glaskolben abgelassen und es wurden 2,0677 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein zunächst klares aber später leicht eintrübendes Produkt mit einer OH-Zahl von 169 mg KOH / g und einer Viskosität von 840 mPas (bei 25 °C) erhalten. Die Polydispersität betrug 1,10.

**Beispiel 13 (Vergleich)**

In einen 2 L Laborautoklaven wurden unter Stickstoffatmosphäre 115,4 g Glycerin und 10,574 g wässrige KOH-Lösung, enthaltend 46,49 Gew.-% KOH, gegeben. Der Inhalt des Autoklaven wurde sodann bei 115 °C unter Rühren (Kreuzbalkenrührer, 200 U/min.) und angelegtem Vakuum bei gleichzeitiger Einleitung von 50 ml Stickstoff pro Minute über einen Zeitraum von 3 h entwässert. Hierbei stellte sich gegen Ende der Entwässerungszeit ein Druck von 100 - 120 mbar ein. Man erwärmte auf 120 °C und dosierte 871,6 g Propylenoxid bei einer Rührerdrehzahl von 800 U/min. über einen Zeitraum von 4,27 h in den Kopfraum des Autoklaven, man begann die Dosierung bei einem absoluten Druck von 0,05 bar. Nach Ende der Propylenoxiddosierung schloss sich eine Nachreaktionszeit von 1,53 h Dauer an. Die zu diesem Zeitpunkt auf Basis der OH-Zahl des Starters, der Startermenge, der Katalysatormenge und der dosierten Propylenoxidmenge berechnete OH-Zahl des Reaktorinhaltes betrug 213 mg KOH / g. Der Reaktordruck wurde sodann mittels Stickstoff auf 2,75 bar eingestellt und es wurden 218,2 g Ethylenoxid ("oxyethyleneinheitenreicher Block") über einen Zeitraum von 1,18 h ebenfalls bei 120 °C in den Kopfraum des Autoklaven dosiert. Nach einer Nachreaktionszeit von 1,03 h Dauer wurde das Produkt im Vakuum (10 mbar) über einen Zeitraum von 120 min. ausgeheizt. Nach Abkühlen auf 80 °C wurden 120 mL destilliertes Wasser und 35,458 g einer 12,114 gew.-%igen wässrigen Schwefelsäurelösung zugegeben und bei 80 °C über einen Zeitraum von 60 min. verrührt. Das entstandene Gemisch wurde in einen Glaskolben abgelassen und es wurden 2,0287 g IRGANOX® 1076 zugegeben. Danach wurde das Produkt 1 h bei 18 mbar (Wasserstrahlvakuum) und anschließend bei 110 °C über einen Zeitraum von 3 h bei 1 mbar entwässert. Nach Filtration über ein Tiefenfilter (T 750, Fa. Seitz) wurde ein klares, lagerstabiles Produkt mit einer OH-Zahl von 176 mg KOH / g erhalten.

In diesem Vergleichsbeispiel wurden die kritischen Parameter nach Ungleichung (1) gemäß den Werten des Vergleichsbeispiels 9 eingestellt, es wurde jedoch ein bei Raumtemperatur flüssiger Starter (Glycerin) eingesetzt.

In Tabelle 1 und Tabelle 2 sind die Versuchsergebnisse zusammengefasst:

Tabelle 1:

| Beispiel | $x_n(EO)$ [mol] | $x(CAT)$ [mol] | $t_n$[h] | $T_n$[°C] | $T_a$[°C] | OH-Zahl Produkt [mg KOH / g] | Viskosität bei 25 °C [mPas] | Polydispersität | Aussehen |
|---|---|---|---|---|---|---|---|---|---|
| 1 | 4,969 | 0,086 | 3,2 | 130 | 130 | 182 | 808 | 1,09 | klar |
| 2 | 4,953 | 0,043 | 3,2 | 150 | 150 | 172 | 843 | 1,03 | klar |
| 3 | 4,933 | 0,087 | 3,2 | 150 | 120 | 171 | 847 | 1,03 | klar |
| 4 | 4,976 | 0,043 | 3,2 | 150 | 120 | 171 | 836 | 1,03 | klar |
| 5 | 4,951 | 0,086 | 3,2 | 150 | 150 | 182 | 773 | 1,18 | klar |
| 6 | 4,958 | 0,176 | 1,1 | 150 | 150 | 181 | 773 | 1,15 | klar |
| 7 | 5,153 | 0,024 | 6,1 | 150 | 150 | 104 | 656 | 1,03 | klar |
| 8 | 4,563 | 0,071 | 1,8 | 150 | 150[#] | 290 | 2470 | 1,03 | klar |
| 9[##] | 4,978 | 0,087 | 1,3 | 120 | 120 | 162 | Nicht bestimmbar | 1,10 | fest |
| 10[##] | 4,933 | 0,086 | 0,63 | 120 | 120 | 164 | 1700 | 1,04 | trüb |
| 11[##] | 4,949 | 0,086 | 0,80 | 130 | 130 | 167 | Nicht bestimmbar | 1,03 | fest |
| 12[##] | 4,933 | 0,086 | 0,92 | 150 | 150 | 169 | 840 | 1,10 | trüb |
| 13[##] | 4,953 | 0,088 | 1,2 | 120 | 120 | 176 | 250 | 1,05 | klar |

[#]: Reaktionstemperatur bis zum Erreichen der Ziel-OH-Zahl des Polyetherpolyols
[##]: Vergleichsbeispiele

**Tabelle 2:**

| Beispiel | $(T_n * x(cat) * t_n) / x_n(EO)$ [°C * h] | $(T_a * x(cat))/m_a$ [°C * mol / kg][#] |
|---|---|---|
| 1 | 7,2 | 11,4 |
| 2 | 4,2 | 6,6 |
| 3 | 8,3 | 10,6 |
| 4 | 4,2 | 5,3 |
| 5 | 8,4 | 13,2 |
| 6 | 6,0 | 26,9 |
| 7 | 4,2 | 6,4 |
| 8 | 4,3 | 8,6[##] |
| 9 (Vergleich) | 2,7 | 10,6 |
| 10 (Vergleich) | 1,3 | 10,5 |
| 11 (Vergleich) | 1,8 | 11,5 |
| 12 (Vergleich) | 2,4 | 13,2 |
| 13 (Vergleich) | 2,5 | 6,5 |
| [#] bei Durchlaufen des OH-Zahlbereiches von 850 bis 215 mg KOH / g <br> [##] bei Durchlaufen des OH-Zahlbereiches von 850 mg KOH / g bis zur Ziel-OH-Zahl des Polyetherpolyols | | |

**Patentansprüche**

1. Verfahren zur Herstellung von Polyetherpolyolen mit n Blöcken $E_n$, jeweils umfassend wenigstens 75 Gew.-% Oxyethyleneinheiten, und m von $E_n$ verschiedenen Blöcken Am, mit n und m als natürlichen Zahlen von 1 bis 10, wobei bei dem Verfahren wenigstens eine Starterverbindung mit zumindest einem Zerewitinoff-aktiven Wasserstoffatom unter Zusatz eines basischen Katalysators mit Ethylenoxid sowie Propylenoxid umgesetzt wird, wobei keine Lösungsmittel verwendet werden, und die wenigstens eine Starterverbindung einen Schmelzpunkt von $\geq$ 40 °C aufweist,

**dadurch gekennzeichnet, dass**

die Umsetzung mit Ethylenoxid während der Dosierung des Blockes oder der Blöcke $E_n$ unter den Bedingungen der folgenden Ungleichung erfolgt:

$$\frac{T_n * x(cat) * t_n}{x_n(EO)} > 2{,}9$$

$$\text{in } [°C * h] \qquad\qquad (1)$$

wobei

$T_n$, als mittlere Reaktionstemperatur in °C während der Dosierung des Ethylenoxids für den Block $E_n$,
$x(cat)$, als die Stoffmenge an eingesetztem Katalysator in mol,
$t_n$, als die Dosierzeit für das Ethylenoxid des Blocks $E_n$ in Stunden,
$x_n(EO)$, als die eindosierte Stoffmenge an Ethylenoxid für den Block $E_n$ in mol,
n wie vorstehend

definiert sind, und
wobei alle Blöcke Am höchstens 40 Gew.-% Oxyethyleneinheiten umfassen,
und wobei der Gesamtgehalt an Oxyethyleneinheiten in den Polyetherpolyolen, bezogen auf die Gesamtmasse aller eindosierten Alkylenoxide, von 5 bis zu 45 Gew.-% beträgt.

**2.** Verfahren gemäß Anspruch 1, **dadurch gekennzeichnet, dass** die OH-Zahl während der Umsetzung der wenigstens einen Starterverbindung mit Ethylenoxid sowie Propylenoxid abnimmt und dabei folgende Ungleichung (3) gilt:

$$T_a * x(cat)/m_a < 12{,}5$$

$$\text{in } [°C * \text{mol/kg}] \qquad (3)$$

mit

$T_a$ als mittlere Reaktionstemperatur in °C während der Umsetzung der wenigstens einen Starterverbindung,
$x(cat)$ als die Stoffmenge an eingesetztem Katalysator in mol und
$m_a$ als Masse des Ansatzes in kg nach erfolgter Umsetzung der wenigstens einen Starterverbindung,

wobei die Ungleichung (3) insbesondere ab einer OH-Zahl von 1000 mg KOH / g oder weniger gilt, vorzugsweise ab 850 mg KOH / g oder weniger.

**3.** Verfahren nach Anspruch 2, **dadurch gekennzeichnet, dass** die Ungleichung (3) bis zu einer OH-Zahl von 215 mg KOH / g gilt und $m_a$ die Masse des Ansatzes in kg bei Passieren der OH-Zahl 215 mg KOH / g bezeichnet.

**4.** Verfahren gemäß einem der vorstehenden Ansprüche, **dadurch gekennzeichnet, dass** die OH-Zahlen der Polyetherpolyole von 28 mg KOH / g bis 500 mg KOH / g betragen.

**5.** Verfahren gemäß einem der vorstehenden Ansprüche, **dadurch gekennzeichnet, dass** die Polyetherpolyole neben den Struktureinheiten, die auf den Starter zurückgehen, ausschließlich aus den Blöcken $E_n$ und Am bestehen.

**6.** Verfahren gemäß einem der vorstehenden Ansprüche, **dadurch gekennzeichnet, dass** wenigstens einer, vorzugsweise alle Blöcke Am höchstens 25 Gew.-% Oxyethyleneinheiten umfassen.

**7.** Verfahren gemäß einem der vorstehenden Ansprüche, **dadurch gekennzeichnet, dass** die Polyetherpolyole bis zu 30 Gew.-%, Oxyethyleneinheiten, bezogen auf die Gesamtmasse aller eindosierten Alkylenoxide, enthalten.

**8.** Verfahren gemäß einem der vorstehenden Ansprüche, **dadurch gekennzeichnet, dass** zumindest eine weitere Starterverbindung mit einem Schmelzpunkt < 40 °C in einem Anteil bis zu 30 Gew.-%, bezogen auf die Gesamtmasse aller Starterverbindungen, eingesetzt wird.

**9.** Verfahren gemäß einem der vorstehenden Ansprüche, **dadurch gekennzeichnet, dass** der Katalysator Kaliumhydroxid, ist, und/ oder dass der Katalysator in einer auf die Endproduktmenge bezogenen Konzentration von 0,004 bis 1,0 Gew.-% verwendet wird, insbesondere von 0,02 bis 1,0 Gew.-%.

**10.** Verfahren gemäß einem der vorstehenden Ansprüche, **dadurch gekennzeichnet, dass** mindestens ein Block $E_n$ wenigstens 90 Gew.-% Oxyethyleneinheiten enthält.

**11.** Verfahren gemäß einem der vorstehenden Ansprüche, **dadurch gekennzeichnet, dass** ein Block $E_n$ ein Endblock ist.

**12.** Verfahren gemäß einem der vorstehenden Ansprüche, **dadurch gekennzeichnet, dass** die Umsetzung der wenigstens einen Starterverbindung mit Ethylenoxid sowie Propylenoxid bei einer Temperatur von 70 bis 170 °C erfolgt, insbesondere von 100 bis 160 °C.

**13.** Verfahren gemäß einem der vorstehenden Ansprüche, **dadurch gekennzeichnet, dass** als Starterverbindung Pentaerythrit, Saccharose, Trimethylolpropan und / oder Sorbit eingesetzt werden.

**Claims**

**1.** Process for preparing polyether polyols having n $E_n$ blocks each comprising at least 75% by weight of oxyethylene units, and m Am blocks different from $E_n$, where n and m are each natural numbers from 1 to 10, at least one starter compound having at least one Zerevitinov-active hydrogen atom being reacted in the process with ethylene oxide and propylene oxide with addition of a basic catalyst, without using any solvents, and the at least one starter compound having a melting point of $\geq 40°C$, **characterized in that**
the reaction with ethylene oxide is effected during the metered addition of the $E_n$ block(s) under the conditions of the following inequation:

$$\frac{T_n * x(cat) * t_n}{x_n(EO)} > 2.9$$

in [°C * h]

(1)

where

$T_n$, is defined as the mean reaction temperature in °C during the metered addition of the ethylene oxide for the $E_n$ block,
x(cat) is defined as the amount of catalyst used in mol,
$t_n$ is defined as the metering time for the ethylene oxide in the $E_n$ block in hours,
$x_n(EO)$ is defined as the amount of ethylene oxide metered in for the $E_n$ block in mol,
n is as defined above, and

where all $A_m$ blocks comprise not more than 40% by weight of oxyethylene units,
and where the total content of oxyethylene units in the polyether polyols, based on the total mass of all the alkylene oxides metered in, is from 5 up to 45% by weight.

2. Process according to Claim 1, **characterized in that** the OH number during the reaction of the at least one starter compound with ethylene oxide and propylene oxide decreases and the following inequation (3) applies:

$$T_a * x(cat)/m_a < 12.5$$

in [°C * mol/kg]

(3)

with

$T_a$ as the mean reaction temperature in °C during the reaction of the at least one starter compound,
x(cat) as the amount of catalyst used in mol and
$m_a$ as the mass of the mixture in kg on completion of conversion of the at least one starter compound,

where the inequation (3) applies particularly from an OH number of 1000 mg KOH/g or less, preferably from 850 mg KOH/g or less.

3. Process according to Claim 2, **characterized in that** the inequation (3) applies up to an OH number of 215 mg KOH/g and $m_a$ denotes the mass of the mixture in kg when the OH number of 215 mg KOH/g is passed.

4. Process according to any of the preceding claims, **characterized in that** the OH numbers of the polyether polyols are from 28 mg KOH/g to 500 mg KOH/g.

5. Process according to any of the preceding claims, **characterized in that** the polyether polyols, aside from the structural units originating from the starter, consist exclusively of the $E_n$ and $A_m$ blocks.

6. Process according to any of the preceding claims, **characterized in that** at least one $A_m$ block, preferably all $A_m$ blocks, comprise(s) not more than 25% by weight of oxyethylene units.

7. Process according to any of the preceding claims, **characterized in that** the polyether polyols contain up to 30% by weight of oxyethylene units, based on the total mass of all the alkylene oxides metered in.

8. Process according to any of the preceding claims, **characterized in that** at least one further starter compound having a melting point < 40°C is used in a proportion of up to 30% by weight, based on the total mass of all starter compounds.

9. Process according to any of the preceding claims, **characterized in that** the catalyst is potassium hydroxide, and/or **in that** the catalyst is used in a concentration, based on the amount of end product, of 0.004 to 1.0% by weight, especially of 0.02 to 1.0% by weight.

10. Process according to any of the preceding claims, **characterized in that** at least one $E_n$ block contains at least 90% by weight of oxyethylene units.

11. Process according to any of the preceding claims, **characterized in that** one $E_n$ block is an end block.

12. Process according to any of the preceding claims, **characterized in that** the reaction of the at least one starter compound with ethylene oxide and propylene oxide is effected at a temperature of 70 to 170°C, especially of 100 to 160°C.

13. Process according to any of the preceding claims, **characterized in that** the starter compound used is pentaerythritol, sucrose, trimethylolpropane and/or sorbitol.

**Revendications**

1. Procédé de préparation de polyétherpolyols comportant n blocs En, comprenant chacun au moins 75 % en poids de motifs oxyéthylène, et m bloc $A_m$, différents de En, n et m étant des nombres entiers de 1 à 10, procédé dans lequel au moins un composé de départ, comportant au moins un atome d'hydrogène actif selon Zerewitinoff, est mis à réagir, avec addition d'un catalyseur basique, avec de l'oxyde d'éthylène, ainsi que de l'oxyde de propylène, aucun solvant n'étant utilisé, et le ou les composés de départ présentant un point de fusion $\geq$ 40°C,
**caractérisé en ce que**
la réaction avec l'oxyde d'éthylène a lieu pendant le dosage du bloc ou des blocs En, dans les conditions correspondant à l'inéquation suivante :

$$\frac{T_n * x(cat) * t_n}{x_n(EO)} > 2,9 \quad , \text{ en } [°C*h] \quad (1)$$

dans laquelle

$T_n$ est défini comme étant la température moyenne de réaction, en °C, pendant le dosage de l'oxyde d'éthylène pour le bloc $E_n$,
x(cat) est défini comme étant la quantité de matière du catalyseur utilisé, en moles,
$t_n$ est défini comme étant le temps de dosage de l'oxyde d'éthylène du bloc $E_n$, en heures,
$x_n$,(EO) est défini comme étant la quantité de matière dosée d'oxyde d'éthylène, pour le bloc $E_n$, en moles,
n est tel que défini ci-dessus, et

dans lequel tous les blocs $A_m$ comprennent au plus 40 % en poids de motifs oxyéthylène,
et dans lequel la teneur totale des polyétherpolyols en motifs oxyéthylène, rapportée à la masse totale de tous les oxydes d'alkylène dosés, est de 5 à 45 % en poids.

2. Procédé selon la revendication 1, **caractérisé en ce que** l'indice d'OH diminue pendant la réaction du ou des composés de départ avec de l'oxyde d'éthylène, ainsi que de l'oxyde de propylène, l'inéquation (3) ci-après s'appliquant :

$$T_a * x(cat)/m_a < 12,5 \quad \text{ en } [°C*mol/h] \quad (3)$$

dans laquelle

$T_a$ est la température moyenne de réaction, en °C, pendant la réaction du ou des composés de départ, x(cat) est la quantité de matière du catalyseur utilisé, en moles, et

$m_a$ est la masse de la masse réactionnelle, en kg, quand la réaction du ou des composés de départ est terminée,

l'inéquation (3) s'appliquant en particulier à partir d'un indice d'OH de 1000 mg KOH/g ou moins, de préférence à partir de 850 mg KOH/g ou moins.

3. Procédé selon la revendication 2, **caractérisé en ce que** l'inéquation (3) s'applique jusqu'à un indice d'OH de 215 mg KOH/g, et $m_a$ désigne la masse de la masse réactionnelle, en kg, après que l'indice d'OH a dépassé 215 mg KOH/g.

4. Procédé selon l'une des revendications précédentes, **caractérisé en ce que** l'indice d'OH des polyétherpolyols est de 28 mg KOH/g à 500 mg KOH/g.

5. Procédé selon l'une des revendications précédentes, **caractérisé en ce que** les polyétherpolyols sont constitués, en plus des motifs structuraux qui remontent au composé de départ, exclusivement des blocs $E_n$ et $A_m$.

6. Procédé selon l'une des revendications précédentes, **caractérisé en ce qu'**au moins un, de préférence tous les blocs $A_m$ comprennent au plus 25 % en poids de motifs oxyéthylène.

7. Procédé selon l'une des revendications précédentes, **caractérisé en ce que** les polyétherpolyols contiennent jusqu'à 30 % en poids de motifs oxyéthylène, par rapport à la masse totale de tous les oxydes d'alkylène dosés.

8. Procédé selon l'une des revendications précédentes, **caractérisé en ce qu'**au moins un composé de départ supplémentaire est utilisé, présentant un point de fusion < 40°C, selon une proportion allant jusqu'à 30 % en poids par rapport à la masse totale de tous les composés de départ.

9. Procédé selon l'une des revendications précédentes, **caractérisé en ce que** le catalyseur est l'hydroxyde de potassium, et/ou que le catalyseur est utilisé en une concentration, rapportée à la quantité du produit final, de 0,004 à 1,0 % en poids, en particulier de 0,02 à 1,0 % en poids.

10. Procédé selon l'une des revendications précédentes, **caractérisé en ce qu'**au moins un bloc $E_n$ contient au moins 90 % en poids de motifs oxyéthylène.

11. Procédé selon l'une des revendications précédentes, **caractérisé en ce qu'**un bloc $E_n$ est un bloc terminal.

12. Procédé selon l'une des revendications précédentes, **caractérisé en ce que** la réaction du ou des composés de départ avec de l'oxyde d'éthylène, ainsi que de l'oxyde de propylène, est réalisée à une température de 70 à 170°C, en particulier de 100 à 160°C.

13. Procédé selon l'une des revendications précédentes, **caractérisé en ce qu'**on utilise en tant que composé de départ du pentaérythritol, du saccharose, du triméthylolpropane et/ou du sorbitol.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4332936 A
- FR 1285708 A
- US 3190927 A
- DE 1443022 A
- US 4430490 A
- EP 2028211 A
- WO 2009152954 A
- WO 2011039145 A
- WO 2009106244 A
- WO 9820061 A
- US 2004167316 A
- US 4521548 A
- DE 10024313 A
- US 4507475 A
- EP 0693513 A
- EP 1751213 A
- WO 0114456 A
- JP 6157743 A
- WO 9620972 A
- US 3823145 A

**In der Beschreibung aufgeführte Nicht-Patentliteratur**

- **M. IONESCU et al.** *Advances in Urethanes Science and Technology,* 1998, vol. 14, 151-218
- Ullmann's Encyclopedia of Industrial Chemistry. 1992, vol. B4, 167ff
- Handbuch Apparate. Vulkan-Verlag Essen, 1990, 188-208